# EUROPÄISCHE PATENTSCHRIFT

(11) **EP 3 713 618 B1**
(45) Veröffentlichungstag und Bekanntmachung des Hinweises auf die Patenterteilung: **18.03.2026**
(21) Anmeldenummer: 18811744.4
(22) Anmeldetag: 20.11.2018
(51) Int. Cl.: A61M 1/16

(54) **VORRICHTUNG UND VERFAHREN ZUR HERSTELLUNG DES DIALYSATES**
DEVICE AND METHOD FOR THE PREPARATION OF DIALYSATE
DISPOSITIF ET MÉTHODE POUR LA PRÉPARATION DU DIALYSAT

(30) Priorität: 22.11.2017 DE 102017127637
(43) Veröffentlichungstag der Anmeldung: 30.09.2020
(73) Patentinhaber: Vivonic GmbH, 63877 Sailauf (DE)
(72) Erfinder: BESSLER, Patrick, 97837 Erlenbach (DE); EBERLEIN, Stefan, 97204 Höchberg (DE); HEMM, Andreas, 63755 Alzenau (DE)
(74) Vertreter: Lorenz Seidler Gossel Part. mbB
(86) Internationale Anmeldenummer: PCT/EP2018/081979
(87) Internationale Veröffentlichungsnummer: WO 2019/101757

(56) Entgegenhaltungen:
- DE-A1- 102012 004 886
- GB-A- 2 097 285

## Beschreibung

Die Erfindung betrifft eine Vorrichtung und ein Verfahren zur Herstellung einer medizinischen Lösung und vorzugsweise Dialysekonzentratlösung.

Im Stand der Technik sind mehrere Verfahren zur Herstellung von Dialysekonzentratlösungen aus nicht oder nur teilweise gelösten Rohstoffen bekannt. Beispielsweise ist es bekannt, Rohstoffe in einem Ansatzbehälter mit Hilfe eines Rührers in einem Lösungsmittel aufzulösen, typischerweise in RO-Wasser, was auch als Permeat oder Dialysewasser bezeichnet wird. Ferner bekannt ist ein zyklisches Einspülen und Aussaugen von Lösungsmittel direkt in und aus einem Rohstoffbehälter über einen einzigen Anschluss. Dieser Vorgang dauert aber relativ lange und der Auflösungsgrad ist schlecht kontrollierbar. Die Druckschrift GB2097285A offenbart beispielsweise eine Vorrichtung und ein Verfahren zur Herstellung von Dialysat. DE10 2012 004886 offenbart ein zumindest teilweise automatisiertes Verfahren zur Herstellung von Dialysat. Weiterhin ist es bekannt, einen Rohstoffbehälter mit einem Lösungsmittel zu durchströmen. Der Rohstoffbehälter kann beispielsweise zwei Anschlüsse aufweisen und von oben nach unten oder umgekehrt durchströmt werden. Hierbei wird typischerweise kontinuierlich Flüssigkeit von oben oder unten in den Rohstoffbehälter eingespült und unten oder oben abgezogen. In einem derartigen Verfahren gelangt aber ein Teil des Rohstoffs in den Produktbehälter, sofern dies nicht durch gesonderte Maßnahmen verhindert wird. Wenn ungelöster Rohstoff in den Produktbehälter gelangt, besteht das Risiko, dass er ungelöst verbleibt und die Zusammensetzung der fertigen Lösung nicht korrekt ist, d.h. nicht den Vorgaben entspricht. Bei diesen bekannten Verfahren muss der Rohstoffbehälter außerdem als Druckbehälter ausgeführt werden, da der Aufbau eines Überdrucks im Rohstoffbehälter nicht verhindert werden kann.

Aufgabe der Erfindung ist es, eine Vorrichtung und ein Verfahren zur Herstellung einer medizinischen Lösung und vorzugsweise einer Dialysekonzentratlösung bereitzustellen, die es ermöglicht, in beliebigen Rohstoffbehältern untergebrachte Rohstoffe verschiedener Konsistenz bei hoher Mischsicherheit mit einem Lösungsmittel zu vermischen.

Diese Aufgabe wird erfindungsgemäß gelöst durch eine Vorrichtung zur Herstellung einer medizinischen Lösung und vorzugsweise einer Dialysekonzentratlösung, das ein Fluidsystem mit einem Hauptkreislauf und einem damit in Verbindung stehenden Zweigabschnitt aufweist, wobei es sich bei dem Hauptkreislauf um einen Kreislauf handelt, in dem eine Pumpe sowie ein Produktbehälter angeordnet sind und in dem durch Betrieb der Pumpe Flüssigkeit zirkuliert werden kann, wobei im Zweigabschnitt ein Rohstoffbehälter anschließbar ist, wobei der Zweigabschnitt eine Saugleitung umfasst, die von dem anzuschließenden Rohstoffbehälter zu einer Ansaugeinheit des Hauptkreislaufs führt, und wobei die Ansaugeinheit ausgebildet ist, Flüssigkeit zusammen mit noch nicht gelöstem Rohstoff aus der Saugleitung anzusaugen und in eine durch den Hauptkreislauf strömende Flüssigkeit einzutragen, wobei weiterhin zwischen der Ansaugeinheit und dem Produktbehälter eine Feinmischkammer im Hauptkreislauf angeordnet ist, die zwei Anschlüsse und ein zwischen den Anschlüssen angeordnetes Rückhalteelement für noch nicht gelösten Rohstoff aufweist.

Die Vorrichtung eignet sich besonders zur Herstellung von Dialysekonzentratlösungen, ist aber grundsätzlich auch zur Herstellung anderer medizinischer Lösungen geeignet, bei der bestimmte Mengen von Rohstoffen in einer bestimmten Menge eines Lösungsmittels aufgelöst bzw. damit vermischt werden sollen. Bei der hergestellten Lösung kann es sich bereits um eine gebrauchsfertige Lösung oder um ein Konzentrat wie ein Dialysekonzentrat handeln, das in einem weiteren Schritt erneut verdünnt werden muss, um eine gebrauchsfertige Lösung zu erhalten.

Wenn im Hauptkreislauf des Fluidsystems durch den Betrieb der Pumpe Lösungsmittel bzw. entstehende Lösung zirkuliert wird, dann durchläuft die Flüssigkeit dabei den Produktbehälter und die Ansaugeinheit. Die Ansaugeinheit ist im Hauptkreislauf vorzugsweise zwischen der Druckseite der Pumpe und dem Produktbehälter angeordnet. Durch die Ausbildung der Ansaugeinheit derart, dass Flüssigkeit zusammen mit noch nicht gelösten Rohstoff aus dem anzuschließenden Rohstoffbehälter durch die Saugleitung in den Hauptkreislauf gezogen wird, herrscht im Betrieb der Vorrichtung in der Saugleitung ein geringerer Druck als im die Ansaugeinheit umfassenden Bereich des Hauptkreislaufs. Der anhand der Saugleitung angeschlossene Rohstoffbehälter muss daher nicht als Druckbehälter ausgeführt werden, sondern es können auch Beutel oder dergleichen als Rohstoffbehälter verwendet werden. Weiterhin muss der Rohstoffbehälter selbst bei einer Ausführung mit einer starren Wandung nicht druckdicht gegenüber der Umgebung ausgeführt sein, sondern kann zur Belüftung dienende Öffnungen ausweisen, die ggf. verschließbar sind.

In einer Ausführungsform ist vorgesehen, dass das Rückhalteelement ein Filterelement umfasst, das den noch nicht gelösten Rohstoff zurückhält. Dabei kann das Filterelement so angeordnet sein, dass die Flüssigkeit mit dem noch nicht gelösten Rohstoff gegen die Schwerkraft fließen muss, um das Filterelement zu durchdringen. Zusätzlich kann die Feinmischkammer eine Verwirbelungskammer umfassen, die die Flüssigkeit vor dem Erreichen des Filterelements zuerst erreicht und die sich um das Filterelement erstreckt, damit die Flüssigkeit die Auflösung des noch nicht gelösten Rohstoffes durch die entstehende Überströmung unterstützen kann.

Vorzugsweise ist die Feinmischkammer so im Hauptkreislauf angeordnet, dass sie von unten nach oben durchströmt wird. Durch die Anordnung der Feinmischkammer in Strömungsrichtung zwischen der Ansaugeinheit und dem Produktbehälter gelangen Rohstoffe aus dem Rohstoffbehälter nicht über die Saugleitung in den Produktbehälter, ohne dass sie die Feinmischkammer durchlaufen. So wird eine sichere Durchmischung gewährleistet und ein Eintrag von ungelöstem Rohstoff in den Produktbehälter vermieden.

In einer Ausführungsform ist vorgesehen, dass wenigstens zwei in Serie oder parallelgeschaltete derartige Feinmischkammern vorgesehen sind, deren Rückhalteelemente unterschiedliche Charakteristika und vorzugsweise Partikeldurchlässigkeiten aufweisen. Beispielsweise kann vorgesehen sein, dass das Rückhaltevermögen der in Strömungsrichtung nachgelagerten Feinmischkammer feiner ist als das Rückhaltevermögen der in Strömungsrichtung vorgelagerten Feinmischkammer. Diese Anordnung sorgt für eine optimale Auflösung und vermeidet etwaige Verstopfungen des Kreislaufs. Generell ist eine Kombination von mehreren parallel und/oder seriell geschalteten Feinmischkammern mit identischen oder unterschiedlichen Rückhaltevermögen möglich, um die Durchmischung zu optimieren.

In einer Ausführungsform ist vorgesehen, dass der Hauptkreislauf und der Zweigabschnitt so ausgebildet sind, dass ein anzuschließender Rohstoffbehälter über die Saugleitung und die Ansaugeinheit zunächst entgegen der Saugrichtung mit Flüssigkeit befüllt werden kann, bevor der Mischbetrieb beginnt. Dieser Vorgang kann auch bei Bedarf wiederholt werden, z.B. zeit-, füllmengen- oder druckgesteuert. Dadurch wird es möglich, feste oder gallertartige Rohstoffe zu verwenden, die als solche nicht angesaugt werden können. Die in den Rohstoffbehälter eingeleitete Flüssigkeit dient als Trägerflüssigkeit, in der beispielsweise feste Rohstoffe dispergiert werden können. Die rohstoffhaltige Flüssigkeit wird dann durch die Saugleitung abtransportiert.

In einer Ausführungsform ist vorgesehen, dass es sich bei der Ansaugeinheit um eine Venturi-Düse handelt, wobei die Saugleitung in eine Verengung der Leitung des Hauptkreislaufs mündet. In dieser Ausführungsform kann Flüssigkeit zusammen mit noch nicht gelösten Rohstoff aus der Saugleitung und mithin aus dem angeschlossenen Rohstoffbehälter durch Venturi-Injektion in die im Hauptkreislauf zirkulierende Flüssigkeit gesaugt und von dieser mitgenommen werden. Vor dem Mischbetrieb kann der Rohstoffbehälter durch die Venturi-Düse durch einen Druckaufbau mit Hilfe eines geschlossenes Regelventils im Hauptkreislauf entgegen der Saugrichtung durch die Saugleitung befüllt werden.

In einer Ausführungsform ist vorgesehen, dass der Zweigabschnitt ferner eine Spülleitung aufweist, die zwischen der Druckseite der Pumpe und der Ansaugeinheit vom Hauptkreislauf abzweigt und an den Rohstoffbehälter angeschlossen werden kann. In dieser Ausführungsform bildet der Zweigabschnitt einen geschlossenen Bypass zum Hauptkreislauf aus, der über den angeschlossenen Rohstoffbehälter führt. So kann erreicht werden, dass Lösungsmittel bzw. entstehende Dialysekonzentratlösung vom Hauptkreislauf in den angeschlossenen Rohstoffbehälter eingemischt werden kann, um diesen zu durchspülen. Die Spülleitung kann aber insbesondere zur Reinigung des Rohstoffbehälters nach der Verwendung eingesetzt werden.

Beispielsweise kann es sich bei dem Rohstoffbehälter um einen wiederverwendbaren Behälter handeln, der als Druckbehälter ausgebildet sein kann, aber nicht muss. Dieser kann durch einen Befüllanschluss mit einem beliebigen Rohstoff als Ausgangsmaterial für die herzustellende Lösung bzw. das Dialyselösungskonzentrat befüllt werden. Der Rohstoff kann in fester Form, z.B. als Pulver oder Granulat, als fest/flüssiges Gemisch oder als Aufschlämmung vorliegen, wobei er zumindest teilweise nicht gelöste Bestandteile umfasst. Alternativ kann es sich bei dem Rohstoffbehälter um einen Folienbeutel handeln.

Die erfindungsgemäße Vorrichtung ist gleichzeitig geeignet, mit Rohstoffbehältern verwendet zu werden, die nur flüssige Bestandteile aufweisen. Ein besondere Anpassung der Vorrichtung ist für einen solchen Fall nicht erforderlich.

In einer Ausführungsform besteht die Möglichkeit, die Spülleitung und die Saugleitung an einer Koppelstelle kurzzuschließen, so dass die Leitungen nach Abkopplung des Rohstoffbehälters durchspült werden können, beispielsweise um etwaige Konzentratrückstände noch in den Hauptkreislauf einzutragen oder nach Beendigung der Lösungsherstellung zu Reinigungszwecken.

In einer Ausführungsform ist vorgesehen, dass wenigstens ein weiterer Zweigabschnitt vorhanden ist, an den ein weiterer Rohstoffbehälter anschließbar ist und der eine weitere Saugleitung umfasst, die von dem weiteren angeschlossenen Rohstoffbehälter zu einer Ansaugeinheit des Hauptkreislaufs führt. Die weitere Saugleitung ist vorzugsweise parallel zur ersten Saugleitung angeordnet und mündet in dieselbe Ansaugeinheit des Hauptkreislaufs. Es können aber auch mehrere Ansaugeinheiten im Hauptkreislauf vorgesehen sein. In dieser Ausführungsform ist es möglich, mehrere unterschiedliche Rohstoffbehälter an das System anzuschließen, die unterschiedliche Rohstoffe in beispielsweise unterschiedlichen Aggregatzuständen beinhalten. Sofern mehrere Zweigabschnitte vorgesehen sind, können diese identisch oder unterschiedlich ausgebildet sein. Die obigen Ausführungen zu bevorzugten Ausgestaltungen des ersten Zweigabschnitts gelten für jeden weiteren Zweigabschnitt entsprechend.

In einer Ausführungsform ist vorgesehen, dass das Fluidsystem eine Versorgungsleitung aufweist, die in den Hauptkreislauf einmündet. Beispielsweise kann das Fluidsystem über die Versorgungsleitung an eine RO-Wasserquelle angeschlossen werden. Vorzugsweise mündet die Versorgungsleitung zwischen dem Produktbehälter und der Saugseite der Pumpe in den Hauptkreislauf ein.

In einer Ausführungsform ist vorgesehen, dass das Fluidsystem eine Produktleitung aufweist, die vom Hauptkreislauf abzweigt. Beispielsweise können über die Produktleitung Dialysegeräte oder Lösungsbehälter, z.B. zur Lagerung, an die Vorrichtung angeschlossen werden. Vorzugsweise zweigt die Produktleitung zwischen der Druckseite der Pumpe und der Ansaugeinheit vom Hauptkreislauf ab. Die Produktleitung kann auch von der Spülleitung des Zweigabschnitts abzweigen.

In einer weiteren Ausführungsform ist vorgesehen, dass das Fluidsystem eine Heizung aufweist, um darin befindliche Flüssigkeit bzw. Lösung zu erwärmen. Die Heizung ist vorzugsweise im Hauptkreislauf angeordnet und kann beispielsweise zwischen der Druckseite der Pumpe und der Ansaugeinheit liegen.

Das Fluidsystem umfasst vorzugsweise mehrere Ventile, mit denen sich der Durchfluss durch die unterschiedlichen Leitungen des Systems steuern lässt. Beispielsweise können im Hauptkreislauf zwischen Ansaugeinheit und Produktbehälter und/oder in der Spülleitung Regelventile angeordnet sein. In der Saugleitung kann vorzugsweise nahe der Ansaugeinheit ein Regelventil vorgesehen sein. In der Versorgungsleitung und/oder der Produktleitung können Regelventile angeordnet sein.

Die eingangs gestellte Aufgabe wird auch gelöst durch Systeme bestehend aus einer erfindungsmäßen Vorrichtung zur Herstellung einer medizinischen Lösung, vorzugsweise einer Dialysekonzentratlösung, sowie aus einem oder - falls vorgesehen - mehreren angeschlossenen Rohstoffbehältern.

Vor dem eingangs genannten Hintergrund betrifft die Erfindung ferner ein Verfahren zur Herstellung einer medizinischen Lösung und vorzugsweise einer Dialysekonzentratlösung unter Verwendung einer erfindungsgemäßen Vorrichtung, wobei Lösungsmittel bzw. entstehende Lösung durch Betrieb der Pumpe im Hauptkreislauf zirkuliert wird und zeitgleich Rohstoffe durch die Ansaugeinheit aus der Saugleitung angesaugt und in die im Hauptkreislauf zirkulierende Flüssigkeit eingetragen werden. Bei den angesaugten Rohstoffen handelt es sich um flüssige oder in einer Trägerflüssigkeit gelöste oder dispergierte Rohstoffe.

In einer Ausführungsform ist vorgesehen, dass Lösungsmittel bzw. entstehende Lösung aus dem Hauptkreislauf über die Verdünnungsleitung in den Rohstoffbehälter eingetragen, mit den Rohstoffen vermischt und gemeinsam mit den Rohstoffen durch die Saugleitung gezogen wird. Lösungsmittel bzw. entstehende Lösung aus dem Hauptkreislauf wird also als Trägerflüssigkeit für den Rohstoff verwendet. Dies kann einmalig zu Beginn des Herstellungsverfahrens oder auch mehrmalig oder fortlaufend während des Herstellungsverfahrens erfolgen.

Das Verfahren kann insbesondere automatisch oder teilautomatisch durch eine in Mischvorrichtungen vorhandene Steuereinheit durchgeführt werden, die entsprechend konfiguriert ist, die notwendige Aktoren wie Pumpen und Ventile anzusteuern sowie dafür notwendige Messwerte von Sensoren einzulesen.

Vor dem eingangs genannten Hintergrund betrifft die Erfindung weiterhin die Verwendung einer erfindungsgemäßen Vorrichtung zur Herstellung einer medizinischen Lösung und vorzugsweise einer Dialysekonzentratlösung.

Vorteile der Erfindung umfassen die Möglichkeit eines zumindest teilautomatischen Mischprozesses zur vollständigen Auflösung beliebiger Rohstoffe. Aufgrund der Feinmischkammer mit einem Rückhalteelement für noch nicht gelöste Rohstoffe kann eine komplette Auflösung des Rohstoffes sichergestellt werden. Rohstoffeinträge in den Produktbehälter werden vermieden. In einigen Ausführungsbeispielen ist der Mischprozess selbstregulierend, wie nachstehend erläutert werden wird. Es ist möglich, beliebige Rohstoffgebinde wie beispielsweise flexible Einwegbeutel und starre Mehrwegbehälter als Rohstoffquelle anzuschließen. Die Konsistenz des Rohstoffes kann beliebig gewählt werden. Eine Verarbeitung fester Rohstoffe ist ebenso möglich wie eine Verarbeitung gallertiger oder flüssiger Rohstoffe.

Weitere Einzelheiten und Vorteile der Erfindung ergeben sich aus den nachfolgend anhand der Figuren beschriebenen Ausführungsbeispielen. In den Figuren zeigen:
- Figur 1:: einen Flussplan eines Ausführungsbeispiels eines Systems einer erfindungsgemäßen Vorrichtung zur Herstellung einer Dialysekonzentratlösung mit einem angeschlossenen Rohstoffbehälter;
- Figur 2:: einen Schnitt durch ein Ausführungsbeispiel einer Feinmischkammer mit einem als Filterelement ausgebildeten Rückhalteelement;
- Figur 3:: einen Flussplan eines Ausführungsbeispiels eines weiteren Systems aus einer erfindungsgemäßen Vorrichtung zur Herstellung einer Dialysekonzentratlösung mit einem angeschlossenen, als Einwegbeutel ausgestalteten Rohstoffbehälter; und
- Figur 4:: einen Flussplan eines Ausführungsbeispiels eines wiederum anderen Systems einer erfindungsgemäßen Vorrichtung zur Herstellung einer Dialysekonzentratlösung mit zwei angeschlossenen Rohstoffbehältern.

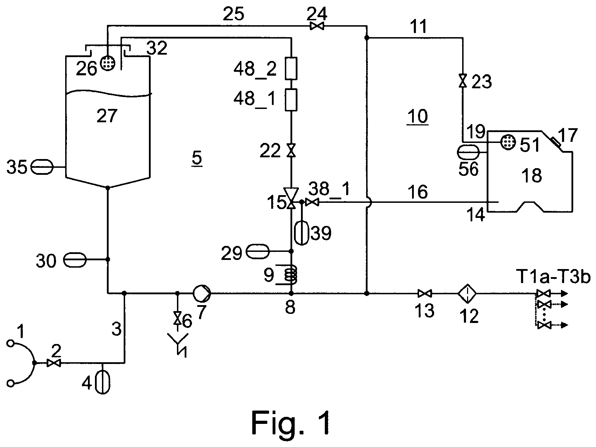

Das in Figur 1 gezeigte Ausführungsbeispiel einer erfindungsgemäßen Vorrichtung umfasst einen Versorgungsanschluss 1 für Lösungsmittel mit einer Versorgungsleitung 3, in der ein Versorgungsventil 2 angeordnet ist. Die Versorgungsleitung 3 mündet in einen Hauptkreislauf 5, in dem unter anderem eine Pumpe 7, ein erstes Regelventil 22 und ein Produktbehälter 27 angeordnet sind.

In einem ersten Vorbereitungsschritt zur Herstellung einer Dialysekonzentratlösung wird bei geöffnetem Ventil 2 RO-Wasser als Lösungsmittel durch den am Versorgungsanschluss 1 anliegenden Ladedruck durch die Versorgungsleitung 3 in den Produktbehälter 27 gefüllt. Die Menge der in den Produktbehälter 27 eingelaufenen Flüssigkeit bzw. dessen Füllstand können anhand des Flussmessers 4 bzw. anhand der Füllstandssensoren 30 und 35 ermittelt werden. Zur Ermittlung des Füllstands im Produktbehälter 27 kann ferner ein in der Figur nicht dargestellter gravimetrischer Sensor vorgesehen sein. Nach Abschluss dieses Vorbereitungsschritts kann das Versorgungsventil 3 geschlossen werden.

Die Vorrichtung umfasst ferner einen Zweigabschnitt 10, der mit dem Hauptkreislauf 5 in Verbindung steht. Der Zweigabschnitt 10 zweigt an einem Verzweigungspunkt 8 auf der Druckseite der Pumpe 7 vom Hauptkreislauf 5 ab und umfasst eine Spülleitung 11, ein zweites Regelventil 23 und eine Saugleitung 16, die ein weiteres Regelventil 38_1 umfasst. Die Saugleitung 16 sowie die Spülleitung 11 sind an einen Rohstoffbehälter 18 anschließbar.

Die Saugleitung 16 mündet an einer als Venturi-Düse ausgestalteten Ansaugeinheit 15 in den Hauptkreislauf 5. Bei dem Rohstoffbehälter 18 handelt es sich in der Ausführungsform gemäß Figur 1 um einen starren und mehrfach verwendbaren Behälter mit einem Anschluss 19 für die Spülleitung 11, einem Anschluss 14 für die Saugleitung 16 und mit einem Befüllanschluss 17 zum Befüllen und ggf. Entleeren des Rohstoffbehälters 18. Des Weiteren ist ein Füllstandssensor 56 zur Ermittlung des Füllstands im Rohstoffbehälter 18 vorgesehen, insbesondere zur Vermeidung eines Überlaufens des Rohstoffbehälters 18.

In einem zweiten Vorbereitungsschritt zur Herstellung einer Dialysekonzentratlösung wird der Rohstoffbehälter 18 an die Vorrichtung angeschlossen. Sollte der Rohstoffbehälter nicht bereits mit Rohstoff befüllt sein, kann er durch den Befüllanschluss 17 mit einer bestimmten Menge eines Rohstoffes befüllt werden. Dabei kann es sich um einen festen Rohstoff, beispielsweise ein Pulver oder ein Granulat, oder ein dickflüssiges, beispielsweise schlammiges, oder dünnflüssiges Konzentrat handeln. Die (Wieder-)Befüllung kann im angeschlossenen oder im getrennten Zustand des Rohstoffbehälters erfolgen.

Bei Verwendung fester Rohstoffe oder dickflüssiger Konzentrate kann in einem dritten Vorbereitungsschritt vorgesehen sein, anhand der Pumpe 7 bei Öffnung des Ventils 38_1 und bei geschlossenem Regelventil 22 einen Teil des Lösungsmittels aus dem Produktbehälter 27 durch die Saugleitung 16 entgegen der Saugrichtung in den Rohstoffbehälter 18 zu pumpen, der zu diesem Zweck durch eine nicht dargestelltes Belüftungsmittel zur Umgebung belüftet ist. Dieser Teil des Lösungsmittels dient als Transportmedium für beispielsweise den festen Rohstoff, der darin, wenngleich in diesem Schritt noch kaum gelöst, zumindest dispergiert und somit leitungsgängig gemacht wird.

Die Reihenfolge der Vorbereitungsschritte ist im Wesentlichen beliebig, mit der einzigen Einschränkung, dass der Produktbehälter 27 mit Lösungsmittel befüllt werden muss, bevor ein Teil des darin befindlichen Lösungsmittels in den Rohstoffbehälter 18 gepumpt wird. So kann beispielsweise vorgesehen sein, Rohstoff erst nach einer teilweisen Befüllung mit Lösungsmittel in den Rohstoffbehälter 18 zu füllen. Die Befüllung des Rohstoffbehälters 18 mit dem Rohstoff kann auch vor der Befüllung des Produktbehälters 27 mit Lösungsmittel erfolgen.

Bei Beginn des Mischvorgangs wird Lösungsmittel aus dem Produktbehälter 27 anhand der Pumpe 7 bei geöffnetem ersten Regelventil 22 im Hauptkreislauf 5 zirkuliert. Dabei wird an der Ansaugeeinheit 15 durch Venturi-Injektion flüssiger bzw. in Flüssigkeit dispergierter und nicht gelöster Rohstoff aus dem angeschlossenen Rohstoffbehälter 18 durch die Saugleitung 16 in das im Hauptkreislauf 5 zirkulierende Lösungsmittel gesaugt und mitgenommen. In Strömungsrichtung hinter der Ansaugeinheit 15 sind im Hauptkreislauf 5 in Serie angeordnete Feinmischkammern 48_1 und 48_2 vorgesehen. In den Feinmischkammern 48_1 und 48_2 wird der noch nicht gelöste Rohstoff im Lösungsmittel gelöst. Die Feinmischkammern 48_1 und 48_2 umfassen jeweils ein für die nicht gelösten Bestandteile des Rohstoffs vorgesehenes Rückhaltelement. Das Vorhandensein von zwei Feinmischkammern 48_1 und 48_2 dient der Optimierung der erfindungsgemäßen Vorrichtung. Das Prinzip der Erfindung erfordert nur das Vorhandensein mindestens einer Feinmischkammer 48_1.

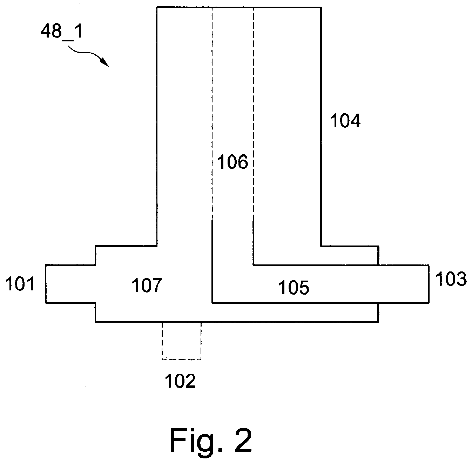

Figur 2 zeigt ein Ausführungsbeispiel für eine solche Feinmischkammer 48_1 bzw.-48_2, bei der das Rückhalteelement als Filterelement ausgebildet ist. Durch den Zulauf 101 der Feinmischkammer wird die Flüssigkeit mit dem zumindest teilweise ungelösten Konzentratrohstoff seitlich unten und bevorzugt mit lateralem Versatz in das Feinmischkammergehäuse 104 eingeströmt und gelangt in eine Verwirbelungskammer 107, die sich um das Filterelement 106 erstreckt. Alternativ kann der Feinmischkammerzulauf auch unten (102) statt seitlich am Feinmischkammergehäuse 104 platziert werden.

Durch die Einströmung der Suspension von seitlich unten oder von unten entsteht im Feinmischkammergehäuse 104 in der Verwirbelungskammer 107 eine Verwirbelung. Um zu dem Filterelement 106 zu gelangen, muss die Flüssigkeit mit dem noch nicht gelösten Rohstoff die Verwirbelungskammer 107 entgegen der Schwerkraft nach oben durchströmen. Nicht gelöste Bestandteile werden dabei durch die Verwirbelung als auch die Schwerkraft bevorzugt in der Verwirbelungskammer 107 gehalten, wo ihre Auflösung durch die Verwirbelung beschleunigt wird. Dennoch bis zum Filterelement 106 gelangende nicht gelöste Bestandteile bleiben am Filterelement 106 zurückgehalten und durch das Überströmen der Flüssigkeit aufgelöst. Die durch das Filterelement 106 durchströmende Flüssigkeit bzw. Lösung wird im Sammelrohr 105 gesammelt und über den Auslauf 103 der Feinmischkammer 48_1 weiter in die Mischleitung der Mischanlage entweder zu einer weiteren Feinmischkammer 48_2 (wie in Figur 1 gezeigt) oder direkt zurück in den Produktbehälter 27 geleitet.

Das Filterelement der zweiten Feinmischkammer 48_2 kann eine feinere Partikeldurchlässigkeit als das Filterelement der ersten Feinmischkammer 48_1 aufweisen, d.h. das Rückhaltevermögen der ersten Feinmischkammer kann kleiner als das der zweiten Feinmischkammer 48_2 sein, um ein stufenweises Auflösen des Rohstoffes zu begünstigen.

Die aus den Feinmischkammern 48_1 und 48_2 austretende Flüssigkeit gelangt zurück in den Produktbehälter 27. Die Filter der Feinmischkammern 48_1 und 48_2 lassen gemäß ihres Rückhaltevermögens Flüssigkeit passieren und halten etwaige ungelöste Rohstoffpartikel in dem erforderlichen Ausmaß zurück. So kann vermieden werden, dass Rohstoffpartikel in den Produktbehälter 27 gelangen.

Zur Beschleunigung und Optimierung des Auflöseprozesses kann das Lösungsmittel vor und während des Lösevorgangs erwärmt werden. Zu diesem Zweck sind im Hauptkreislauf 5 in Strömungsrichtung vor der Ansaugeinheit 15 und den Feinmischkammern 48_1 und 48_2 ein Durchlauferhitzer 9 und ein Temperatursensor 29 angeordnet.

In der Saugleitung 16 sind kurz vor der Einmündung in die Ansaugeinheit 15 ein Regelventil 38_1 und ein Sensor 39 angeordnet, der als Drucksensor ausgebildet sein kann. Der Ssensor 39 dient zur Überwachung des Mischprozesses und zur Füllstandserkennung eines angeschlossenen Rohstoffbehälters 18. Durch das Regelventil 38_1 kann die Saugleitung 16 bei Bedarf unterbrochen werden, z.B. wenn der Rohstoffbehälter 18 leer ist und ein Ansaugen von Luft verhindert werden soll.

Das Lösungsmittel bzw. die entstehende Dialysekonzentratlösung wird so lang im Hauptkreislauf 5 zirkuliert, bis der gesamte im angeschlossenen Rohstoffbehälter 18 befindliche Rohstoff aufgelöst ist. Hierbei kann erforderlich sein, im Verlauf des Auflösungsprozesses mehrmals einen zwischengelagerten Schritt durchzuführen, der dem dritten oben beschriebenen Vorbereitungsschritt entspricht, nämlich anhand der Pumpe 7 bei geeigneter Stellung der Regelventile 22 und 38_1 einen Teil des Lösungsmittels aus dem Produktbehälter 27 durch die Saugleitung 16 des Zweigabschnitts 10 in den angeschlossenen Rohstoffbehälter 18 zu pumpen, um weitere Teile des beispielsweise festen Rohstoffs leitungsgängig zu machen. Der vollständige Verbrauch des Rohstoffs kann anhand einer Zeitsteuerung und/oder anhand des Sensors 39 überwacht werden. Ferner kann ein gravimetrischer oder ein anderweitig geeigneter Sensor am Rohstoffbehälter 18 angeordnet sein.

Nachdem der gesamte im Rohstoffbehälter 18 befindliche Rohstoff verbraucht und der Rohstoffbehälter 18 mit der Ansaugeinheit 15 leergesaugt wurde, kann der Lösungsvorgang und die Zirkulation der nun fertigen Dialysekonzentratlösung im Hauptkreislauf 5 beendet oder zuvor noch für eine gewisse Zeit weiter zirkuliert werden, damit auch in den Feinmischkammern verbleibende Reste von nicht gelöstem Rohstoff aufgelöst werden. Gegebenenfalls kann die Vorrichtung Sensoren zur automatischen Qualitätskontrolle der Dialysekonzentratlösung aufweisen, wie beispielsweise Dichtesensoren, Leitfähigkeitsmesszellen oder Refraktometer.

Das Rückhalten nicht aufgelöster Rohstoffpartikel in einer Feinmischkammer 48_1 bzw. 48_2 ist ein wesentlicher Vorteil der erfindungsgemäßen Lösung. In statischen Mischern, wie sie im Stand der Technik oftmals verwendet wurden, wird der Rohstoff durch Verwirbelung mit dem Lösungsmittel aufgelöst und das Gemisch dann in einen Produktbehälter gepumpt. Nicht aufgelöster Rohstoff gelangt ebenfalls in den Produktbehälter. Im Rahmen des erfindungsgemäßen Herstellungsverfahrens bzw. der erfindungsgemäßen Vorrichtung erfolgt keine Auflösung von Rohstoff im Produktbehälter 27, da der nicht gelöste Rohstoff aufgrund der Feinmischkammer gar nicht erst in den Produktbehälter 27 gelangt.

Wenn das Rückhalteelement in der Feinmischkammer 48_1 bzw. 48_2 als Filterelement ausgebildet ist, kann das System unter Einschluss der Ansaugeinheit 15 ohne weitere Hilfsmittel selbstregulierend arbeiten. Wenn nicht gelöster Rohstoff den Durchfluss durch das Filterelement verringert, vermindert sich der Fluss durch die in diesem Ausführungsbeispiel als Venturi-Düse ausgebildete Ansaugeinheit. Dadurch wird weniger ungelöster Rohstoff angesaugt und in die Feinmischkammer eingetragen, bis sich die verstopfenden Rohstoffanteile durch die überströmende Flüssigkeit lösen und die Verstopfung wieder verringern oder sogar beenden, wodurch der Durchfluss wieder ansteigt. Diese Selbstregulierung begünstigt die Auflösung des Rohstoffs und verhindert gleichzeitig eine signifikante Verstopfung des Rückhalteelements und damit des Flüssigkeitsflusses mit dem Rohstoff.

Die fertige Dialysekonzentratlösung kann anhand der Pumpe 7 durch eine Produktleitung mit einem Filter 12 und einem Transferventil 13 zu unterschiedlichen Abnahmestationen T1a-T3b wie Dialysegeräte oder Lösungsbehältern, z.B. zur Lagerung, gepumpt werden.

Nach Abschluss des Vorgangs kann das System und können alle Flusswege gespült und gegebenenfalls desinfiziert werden. Zu diesem Zweck kann anhand der Pumpe 7 Reinigungsflüssigkeit, beispielsweise RO-Wasser oder mit Peroxiden versetztes RO-Wasser (welches z.B. vom Versorgungsanschluss 1 in den Behälter 27 dosiert bzw. mit Peroxiden versetzt wurde) durch die Leitungen des Systems gepumpt werden. Die Reinigung kann einen angeschlossenen Rohstoffbehälter 18 umfassen, bevor dieser an gleicher oder getrennter Stelle wieder mit Rohstoff befüllt, oder gereinigt entsorgt werden soll. Auch kann die Vorrichtung so ausgebildet sein, dass sie mithilfe eines mit einem Desinfektionsmittel oder -lösung gefüllten Rohstoffbehälters 18 die zur Reinigung benötigte Flüssigkeit selbst wie im Fall der medizinischen Lösung herstellt.

Zum Zwecke der Reinigung ist im Rohstoffbehälter 18 ein mit der Spülleitung 11 verbundener Sprühkopf 51 vorgesehen. Auch im Produktbehälter 27 ist ein Sprühkopf 26 vorgesehen, der an eine Reinigungsleitung 25 mit Reinigungsventil 24 angeschlossen ist. Die Reinigungsleitung 25 zweigt von der Spülleitung 11 des Zweigabschnitts 10 ab. Es kann also eine Spülung des Rohstoffbehälters 18 und des Produktbehälters 27 dadurch vorgenommen werden, dass bei geöffnetem Versorgungsventil 2 und geschlossenem ersten Regelventil 22 von der Pumpe 7 Reinigungsflüssigkeit in die Spülleitung 11 und von dort je nach Stellung des zweiten Regelventils 23 und des Reinigungsventils 24 in den Sprühkopf 51 des Rohstoffbehälters 18 und/oder den Sprühkopf 26 des Produktbehälters 27 gelangt und in den jeweiligen Behälter gesprüht wird. Es ist auch möglich, dass die Reinigungsflüssigkeit vor dem Zirkulieren durch die Pumpe 7 zuerst über den Versorgungsanschluss 1 in den Behälter 27 dosiert wird. Durch Öffnen des ersten Regelventils 22 und Schließen der Regelventile 23 und 24 können die Hauptmischwege mit gereinigt werden. Sollten es die Strömungsverhältnisse zulassen, können die Regelventile 22, 23 und 24 während des Reinigungsvorgangs auch gleichzeitig geöffnet sein. Verbrauchte Reinigungslösung kann durch den Abfluss 6 verworfen werden.

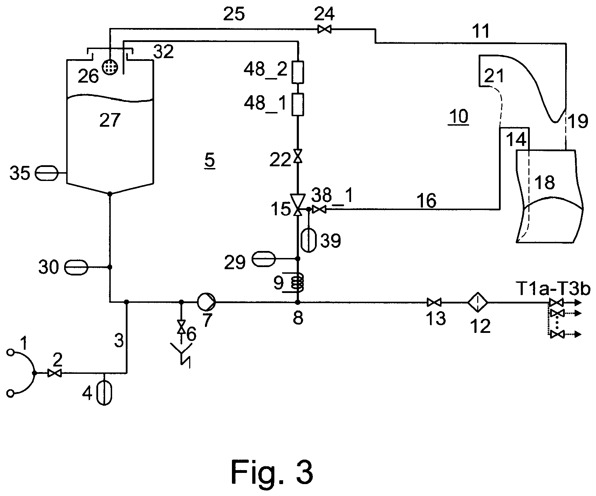

Figur 3 zeigt einen Flussplan eines weiteren Ausführungsfbeispiels einer erfindungsgemäßen Vorrichtung zur Herstellung einer Dialysekonzentratlösung. Der Flussplan ist grundsätzlich dem der Figur 1 ähnlich und es werden nachfolgend lediglich Unterschiede diskutiert.

Im Gegensatz zur Vorrichtung der Figur 1 wird in der Vorrichtung der Figur 3 kein starrer und mehrfach verwendbarer Behälter als Rohstoffbehälter verwendet, sondern ein flexibler Einwegbeutel 18.

Der Einwegbeutel 18 weist einen optionalen Anschluss 19 für die Leitung 11, die hier ggf. zur Entlüftung dient, und einen Anschluss 14 für die Saugleitung 16 des Zweigabschnitts 10 auf. Die Verbindungen zwischen dem optionalen Anschluss 19 und der Leitung 11 einerseits und dem Anschluss 14 und der Saugleitung 16 andererseits sind lösbar. Beispielsweise können die korrespondierenden Anschlüsse mit Schnellverschlusskupplungen ausgestattet sein. Maschinenseitig befindet sich nahe der Anschlussstelle des Einwegbeutels 18 eine Koppelstelle 21, an der sowohl das an den optionalen Anschluss 19 anschließbare Ende der Leitung 11 als auch das an den Anschluss 14 anschließbare Ende der Saugleitung 16 angeschlossen werden können. Die Koppelstelle 21 weist ein Leitungsstück mit zwei Konnektoren, beispielsweise Schnellverschlusskupplungen auf.

Das Ende der Leitung 11 ist an die Koppelstelle 21 angeschlossen oder, im Fall eines optionalen Anschlusses 19, wahlweise an den Anschluss 19. Das Ende der Saugleitung 16 kann wahlweise an den Anschluss 14 oder an die Koppelstelle 21 angeschlossen werden. Sind beide Enden an der Koppelstelle angeschlossen, ergibt sich ein geschlossener Kreislauf unter Aussparung des Beutels 18. Diese Konfiguration soll während der Reinigung des Systems und generell immer dann vorliegen, wenn kein Beutel angeschlossen ist. Ein Anschluss des Endes der Saugleitung 16 an den Anschluss 14 substituiert den zweiten im Zusammenhang mit Figur 1 beschriebenen Vorbereitungsschritt. Diese Konfiguration ist in Figur 3 gezeigt.

Ein Anschluss des Endes der Leitung 11 an den Anschluss 19 kann im Zusammenhang mit dem dritten in Bezug auf Figur 1 beschriebenen Vorbereitungsschritt vorgenommen werden, wenn eine Belüftung des Beutels während eines möglichen Befüllens über die Saugleitung 16 erwünscht ist. Gerade bei Einwegbeuteln kann es aber auch sein, dass der Beutel 18 bereits ausreichend mit Flüssigkeit zum Ausschwemmen der nicht gelösten Rohstoffe herstellerseitig befüllt ist, so dass ein Befüllvorgang mit Flüssigkeit entbehrlich oder teilweise entbehrlich ist.

Im Anschluss wird die medizinische Lösung und vorzugsweise die Dialysekonzentratlösung wie bereits erläutert gemischt und hergestellt.

Im Falle dieses Ausführungsbeispiels kann der Lösungsvorgang beendet werden, nachdem die gesamte im Beutel 18 befindliche rohstoffhaltige Flüssigkeit leergesaugt wurde und restliche in den Feinmischkammern verbliebende Rohstoffmengen aufgelöst wurden.

Für die anschließende Reinigung werden der Beutel 18 abgetrennt und die Saugleitung 16 sowie die Leitung 11 - wenn nicht schon verbunden - über die Koppelstelle 21 kurzgeschlossen. Zur Reinigung der Saugleitung 16 und der Leitungen 11 und 25 müssen die Regelventile 38_1 und 24 geöffnet werden.

Selbstverständlich kann eine erfindungsgemäße Vorrichtung so ausgeführt sein, dass sowohl ein starrer und mehrfach verwendbarer Rohstoffbehälter wie in Figur 1 als auch ein flexibler Einwegbeutel wie in Figur 3 angeschlossen werden können. Hierfür können alternative Anschlüsse und/oder Steckplätze vorgesehen sein.

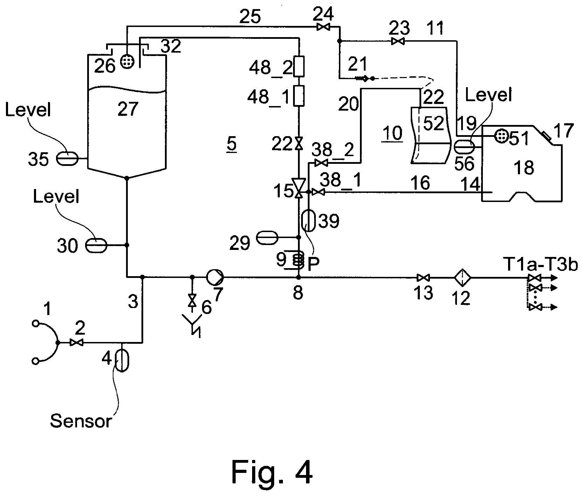

Figur 4 zeigt einen Flussplan eines wiederum anderen Ausführungsbeispiels einer erfindungsgemäßen Vorrichtung. Der Flussplan entspricht im Wesentlichen einer Kombination der in den Figuren 1 und 3 gezeigten Ausführungsbeispiele.

In der Vorrichtung der Figur 4 ist eine zweite Saugleitung 20 mit einem zweiten Regelventil 38_2 vorgesehen, die parallel zur ersten Saugleitung 16 verläuft und am Messpunkt des Sensors 39, zwischen dem Regelventil 38_1 und der Ansaugeinheit 15, mithin also kurz vor der Ansaugeinheit 15 in die erste Saugleitung 16 einmündet. Diese zweite Saugleitung 20 dient dem Anschluss eines zweiten Rohstoffbehälters 52 in Form eines mit einer Flüssigkeit gefüllten Einwegbeutels. In diesem Beutel 52 können ein flüssiges Konzentrat oder eine Wirkstofflösung vorliegen, das oder die zusätzlich zum Rohstoff des Rohstoffbehälters 18 in die Dialysekonzentratlösung eingebracht werden soll. Hierzu ist das Ende der zweiten Saugleitung 20 an den Anschluss 22 des zweiten Rohstoffbehälter 52 anschließbar. Bei Zirkulation des Lösungsmittels bzw. der entstehenden Dialysekonzentratlösung im Hauptkreislauf 5 und Durchströmen der Ansaugeinheit 15 wird dann durch Venturi-Injektion nicht nur rohstoffhaltige Flüssigkeit durch die Saugleitung 16, sondern auch ein zusätzlicher Rohstoff durch die zweite Saugleitung 20 (beispielsweise alternierend oder gleichzeitig) in das im Hauptkreislauf 5 zirkulierende Lösungsmittel gesaugt und mitgenommen.

Im Falle dieser Ausführungsform kann der Lösungsvorgang beendet werden, nachdem die gesamte im Behälter 18 befindliche rohstoffhaltige Flüssigkeit sowie der gesamte im Beutel 52 befindliche zusätzliche Inhaltsstoff leergesaugt und restliche in den Feinmischkammern verbliebende Rohstoffmengen aufgelöst wurden.

Ist kein zusätzlicher Rohstoffbehälter 52 angeschlossen, beispielsweise bei Nichtverwendung oder bei Spülung des Kreislaufs, kann das Ende der zweiten Saugleitung 20 an eine von der Spülleitung 11 abgehende Koppelstelle 21 mit Rückschlagventil angeschlossen werden, die einen geeigneten Konnektor, beispielsweise eine Schnellverschlusskupplung umfasst. Im Falle des Ausführungsbeispiels von Figur 4 kann weiterhin eine Entlüftungsmöglichkeit des Behälters 52 über einen Anschluss wie im Fall des Ausführungsbeispiels von Figur 3 vorgesehen sein, was in Figur 4 nicht näher dargestellt ist.

## Patentansprüche

1. Vorrichtung zur Herstellung einer medizinischen Lösung und vorzugsweise einer Dialysekonzentratlösung, die ein Fluidsystem mit einem Hauptkreislauf (5) und einem damit in Verbindung stehenden Zweigabschnitt (10) aufweist, wobei es sich bei dem Hauptkreislauf (5) um einen Kreislauf handelt, in dem eine Pumpe (7) sowie ein Produktbehälter (27) angeordnet sind und in dem durch Betrieb der Pumpe (7) Flüssigkeit zirkuliert werden kann, und wobei im Zweigabschnitt (10) ein Rohstoffbehälter mit einem zumindest teilweise nicht in Lösung befindlichen Rohstoff (18) angeschließbar ist, und der Zweigabschnitt (10) eine Saugleitung (16) umfasst, die von dem anzuschließenden Rohstoffbehälter (18) zu einer Ansaugeinheit (15) des Hauptkreislaufs (5) führt, wobei die Ansaugeinheit (15) ausgebildet ist, Flüssigkeit zusammen mit noch nicht gelöstem Rohstoff aus der Saugleitung (16) anzusaugen und in eine durch den Hauptkreislauf (5) strömende Flüssigkeit einzutragen,
**dadurch gekennzeichnet,**
**dass** zwischen der Ansaugeinheit (15) und dem Produktbehälter (27) eine Feinmischkammer (48_1, 48_2) im Hauptkreislauf (5) angeordnet ist, die zwei Anschlüsse und ein zwischen den Anschlüssen angeordnetes Rückhalteelement für noch nicht gelösten Rohstoff aufweist und,
**dass** die Vorrichtung weiterhin eine Steuereinheit aufweist, die entsprechend konfiguriert ist, ein Verfahren zur Herstellung einer medizinischen Lösung automatisch oder teilautomatisch durchzuführen, wobei Lösungsmittel bzw. entstehende Lösung durch den Betrieb der Pumpe (7) im Hauptkreislauf (5) zirkuliert wird und zeitgleich Rohstoffe durch die Ansaugeinheit (15) aus der Saugleitung (16) angesaugt und in die im Hauptkreislauf (5) zirkulierende Flüssigkeit eingetragen werden, und wobei die Steuereinheit weiter entsprechend konfiguriert ist, die notwendigen Aktoren, inbesondere Pumpen und Ventile, anzusteuern sowie dafür notwendige Messwerte von Sensoren einzulesen.

2. Vorrichtung nach Anspruch 1, **dadurch gekennzeichnet, dass** das Rückhalteelement ein Filterelement umfasst, das den noch nicht gelösten Rohstoff zurückhält.

3. Vorrichtung nach Anspruch 2, **dadurch gekennzeichnet, dass** das Filterelement so angeordnet ist, dass die Flüssigkeit mit dem noch nicht gelösten Rohstoff gegen die Schwerkraft fließen muss, um das Filterelement zu durchdringen.

4. Vorrichtung nach Anspruch 3, dadurch gekannzeichnet, dass die Feinmischkammer (48_1, 48_2) eine Verwirbelungskammer umfasst, die die Flüssigkeit vor dem Erreichen des Filterelements zuerst erreicht und die sich um das Filterelement erstreckt.

5. Vorrichtung nach einem der vorhergehenden Ansprüche, **dadurch gekennzeichnet, dass** wenigstens zwei in Serie oder parallel geschaltete derartige Feinmischkammern (48_1, 48_2) vorgesehen sind, deren Rückhalteelemente unterschiedliche Charakteristika und vorzugsweise Partikeldurchlässigkeiten aufweisen.

6. Vorrichtung nach einem der vorhergehenden Ansprüche, **dadurch gekennzeichnet, dass** es sich bei der Ansaugeinheit (15) um eine aktive Ansaugeinheit, insbesondere eine Pumpe, oder eine passive Ansaugeinheit handelt.

7. Vorrichtung nach einem der vorhergehenden Ansprüche, **dadurch gekennzeichnet, dass** es sich bei der Ansaugeinheit (15) um eine Venturi-Düse handelt, wobei die Saugleitung (16) in eine Verengung der Leitung des Hauptkreislaufs (5) mündet.

8. Vorrichtung nach einem der vorhergehenden Ansprüche, **dadurch gekennzeichnet, dass** der Zweigabschnitt (10) ferner eine Spülleitung (11) aufweist, die zwischen der Druckseite der Pumpe (7) und der Ansaugeinheit (15) vom Hauptkreislauf (5) abzweigt und in den Rohstoffbehälter (18) anschließbar ist.

9. Vorrichtung nach einem der vorhergehenden Ansprüche, **dadurch gekennzeichnet, dass** wenigstens ein weiterer Zweigabschnitt vorhanden ist, an den ein weiterer Rohstoffbehälter (52) anschließbar ist und der eine weitere Saugleitung (20) umfasst, die von dem weiteren anzuschließenden Rohstoffbehälter (52) zu einer Ansaugeinheit (15) des Hauptkreislaufs (5) führt.

10. System aus einer Vorrichtung nach einem der vorhergehenden Ansprüche und einem angeschlossenen Rohstoffbehälter (18).

11. System aus einer Vorrichtung nach Anspruch 9 oder 10 mit wenigstens einem weiteren angeschlossenen Rohstoffbehälter (52).

12. Verfahren zur Herstellung einer medizinischen Lösung und vorzugsweise einer Dialysekonzentratlösung unter Verwendung einer Vorrichtung nach einem der vorhergehenden Ansprüche,
**dadurch gekennzeichnet,**
**dass** Lösungsmittel bzw. entstehende Lösung durch den Betrieb der Pumpe (7) im Hauptkreislauf (5) zirkuliert wird und zeitgleich Rohstoffe durch die Ansaugeinheit (15) aus der Saugleitung (16) angesaugt und in die im Hauptkreislauf (5) zirkulierende Flüssigkeit eingetragen werden, und
**dass** das Verfahren automatisch oder teilautomatisch durch eine in einer Mischvorrichtung vorhandene Steuereinheit durchgeführt wird, die entsprechend konfiguriert ist, die notwendigen Aktoren, inbesondere Pumpen und Ventile, anzusteuern sowie dafür notwendige Messwerte von Sensoren einzulesen.

13. Verfahren nach Anspruch 12, **dadurch gekennzeichnet, dass** Lösungsmittel bzw. entstehende Lösung aus dem Hauptkreislauf (5) über die Verdünnungsleitung (11) in den Rohstoffbehälter (18) eingetragen, mit den Rohstoffen vermischt und gemeinsam mit den Rohstoffen durch die Saugleitung (16) gezogen wird.

14. Verfahren nach Anspruch 12 oder 13, **dadurch gekennzeichnet, dass** die Herstellung der Lösung mit Hilfe der im Hauptkreislauf angeordneten Feinmischkammer (48_1, 48_2) und des darin enthaltenen Rückhalteelementes selbstregulierend erfolgt.

15. Verwendung eines Systems nach einem der Anspruchs 10 oder 11 zur Herstellung einer medizinischen Lösung und vorzugsweise einer Dialysekonzentratlösung.

## Claims

1. Apparatus for preparing a medical solution and preferably a dialysis concentrate solution, comprising a fluid system having a main circuit (5) and a branch section (10) in communication therewith, wherein the main circuit (5) is a circuit in which a pump (7) and a product container (27) are arranged and in which liquid can be circulated by operation of the pump (7), and wherein a raw material container having a raw material (18) that is at least partly not dissolved is connectable in the branch section (10), and wherein the branch section (10) comprises a suction line (16) that leads from the raw material container (18) to be connected to a suction unit (15) of the main circuit (5), wherein the suction unit (15) is configured to suck liquid together with raw material not yet dissolved out of the suction line (16) and to introduce it into a liquid flowing through the main circuit (5),
**characterized in that**
a fine mixing chamber (48_1, 48_2) having two connectors and a retention element for undissolved raw material arranged between the connectors is arranged in the main circuit (5) between the suction unit (15) and the product container (27), and
the apparatus furthermore comprises a control unit that is configured to automatically or semi-automatically carry out a method for preparing a medical solution, wherein solvent or produced solution is circulated in the main circuit (5) by operation of the pump (7) and raw materials are simultaneously sucked out of the suction line (16) by the suction unit (15) and are introduced into the liquid circulating in the main circuit (5), and wherein the control unit is further configured to actuate the necessary actuators, in particular pumps and valves, and to read in the measurement values required therefor from sensors.

2. Apparatus according to claim 1, **characterized in that** the retention element comprises a filter element that retains the undissolved raw material.

3. Apparatus according to claim 2, **characterized in that** the filter element is arranged such that the liquid containing the undissolved raw material has to flow against gravity in order to pass through the filter element.

4. Apparatus according to claim 3, **characterized in that** the fine mixing chamber (48_1, 48_2) comprises a swirl chamber that the liquid reaches first before reaching the filter element and that extends around the filter element.

5. Apparatus according to one of the preceding claims, **characterized in that** at least two such fine mixing chambers (48_1, 48_2) are provided which are connected in series or in parallel and whose retention elements have different characteristics and preferably different particle permeabilities.

6. Apparatus according to one of the preceding claims, **characterized in that** the suction unit (15) is an active suction unit, in particular a pump, or a passive suction unit.

7. Apparatus according to one of the preceding claims, **characterized in that** the suction unit (15) is a Venturi nozzle, wherein the suction line (16) opens into a narrowing portion of the line of the main circuit (5).

8. Apparatus according to one of the preceding claims, **characterized in that** the branch section (10) furthermore comprises a flushing line (11) that branches off from the main circuit (5) between the pressure side of the pump (7) and the suction unit (15) and is connectable to the raw material container (18).

9. Apparatus according to one of the preceding claims, **characterized in that** at least one further branch section is present to which a further raw material container (52) is connectable and that comprises a further suction line (20) that leads from the further raw material container (52) to a suction unit (15) of the main circuit (5).

10. System comprising an apparatus according to one of the preceding claims and a connected raw material container (18).

11. System comprising an apparatus according to claim 9 or claim 10 with at least one further connected raw material container (52).

12. Method for preparing a medical solution and preferably a dialysis concentrate solution using an apparatus according to one of the preceding claims, **characterized in that**
solvent or produced solution is circulated in the main circuit (5) by operation of the pump (7) and raw materials are simultaneously sucked out of the suction line (16) by the suction unit (15) and introduced into the liquid circulating in the main circuit (5), and
the method is carried out automatically or semi-automatically by a control unit provided in a mixing apparatus, which control unit is configured to actuate the necessary actuators, in particular pumps and valves, and to read in the measurement values required therefor from sensors.

13. Method according to claim 12, **characterized in that** solvent or produced solution is introduced from the main circuit (5) via the dilution line (11) into the raw material container (18), mixed with the raw materials, and drawn through the suction line (16) together with the raw materials.

14. Method according to claim 12 or 13, **characterized in that** the preparation of the solution takes place in a self-regulating manner with the aid of the fine mixing chamber (48_1, 48_2) arranged in the main circuit and the retention element contained therein.

15. Use of a system according to claim 10 or 11 for preparing a medical solution and preferably a dialysis concentrate solution.

## Revendications

1. Dispositif destiné à la préparation d'une solution médicale et de préférence d'une solution de concentré de dialyse, comprenant un système de fluide comportant un circuit principal (5) et une section de dérivation (10) en communication avec celui-ci, le circuit principal (5) étant un circuit dans lequel sont disposés une pompe (7) et un récipient de produit (27) et dans lequel un liquide peut être mis en circulation par le fonctionnement de la pompe (7), et la section de dérivation (10) étant apte à recevoir un récipient de matière première comportant une matière première (18) au moins partiellement non dissoute, et la section de dérivation (10) comprenant une conduite d'aspiration (16) qui s'étend du récipient de matière première (18) à raccorder jusqu'à une unité d'aspiration (15) du circuit principal (5), l'unité d'aspiration (15) étant conçue pour aspirer du liquide conjointement avec de la matière première encore non dissoute hors de la conduite d'aspiration (16) et pour l'introduire dans un liquide s'écoulant dans le circuit principal (5),
**caractérisé en ce que**
une chambre de mélange fin (48_1, 48_2) comportant deux raccords et un élément de retenue pour la matière première non dissoute, disposé entre les raccords, est agencée dans le circuit principal (5) entre l'unité d'aspiration (15) et le récipient de produit (27), et
le dispositif comprend en outre une unité de commande configurée pour exécuter automatiquement ou semi-automatiquement un procédé de préparation d'une solution médicale, dans lequel un solvant ou la solution en cours de formation est mis en circulation dans le circuit principal (5) par le fonctionnement de la pompe (7) et, simultanément, des matières premières sont aspirées hors de la conduite d'aspiration (16) par l'unité d'aspiration (15) et introduites dans le liquide circulant dans le circuit principal (5), l'unité de commande étant en outre configurée pour piloter les actionneurs nécessaires, notamment des pompes et des vannes, et pour lire les valeurs de mesure nécessaires provenant de capteurs.

2. Dispositif selon la revendication 1, **caractérisé en ce que** l'élément de retenue comprend un élément filtrant qui retient la matière première encore non dissoute.

3. Dispositif selon la revendication 2, **caractérisé en ce que** l'élément filtrant est disposé de telle sorte que le liquide contenant la matière première encore non dissoute doit s'écouler à l'encontre de la gravité pour traverser l'élément filtrant.

4. Dispositif selon la revendication 3, **caractérisé en ce que** la chambre de mélange fin (48_1, 48_2) comprend une chambre de tourbillonnement que le liquide atteint en premier avant d'atteindre l'élément filtrant et qui s'étend autour de l'élément filtrant.

5. Dispositif selon l'une des revendications précédentes, **caractérisé en ce qu'**au moins deux chambres de mélange fin de ce type (48_1, 48_2) sont prévues, montées en série ou en parallèle, dont les éléments de retenue présentent des caractéristiques différentes et de préférence des perméabilités aux particules différentes.

6. Dispositif selon l'une des revendications précédentes, **caractérisé en ce que** l'unité d'aspiration (15) est une unité d'aspiration active, en particulier une pompe, ou une unité d'aspiration passive.

7. Dispositif selon l'une des revendications précédentes, **caractérisé en ce que** l'unité d'aspiration (15) est une buse de Venturi, la conduite d'aspiration (16) débouchant dans un rétrécissement de la conduite du circuit principal (5).

8. Dispositif selon l'une des revendications précédentes, **caractérisé en ce que** la section de dérivation (10) comprend en outre une conduite de rinçage (11) qui se détache du circuit principal (5) entre le côté pression de la pompe (7) et l'unité d'aspiration (15) et qui peut être raccordée au récipient de matière première (18).

9. Dispositif selon l'une des revendications précédentes, **caractérisé en ce qu'**au moins une autre section de dérivation est prévue, à laquelle un autre récipient de matière première (52) peut être raccordé et qui comprend une autre conduite d'aspiration (20) s'étendant du récipient de matière première supplémentaire (52) jusqu'à une unité d'aspiration (15) du circuit principal (5).

10. Système comprenant un dispositif selon l'une des revendications précédentes et un récipient de matière première (18) raccordé.

11. Système comprenant un dispositif selon la revendication 9 ou la revendication 10 et au moins un autre récipient de matière première (52) raccordé.

12. Procédé de préparation d'une solution médicale et de préférence d'une solution de concentré de dialyse à l'aide d'un dispositif selon l'une des revendications précédentes,
**caractérisé en ce que**
un solvant ou la solution en cours de formation est mis en circulation dans le circuit principal (5) par le fonctionnement de la pompe (7) et, simultanément, des matières premières sont aspirées hors de la conduite d'aspiration (16) par l'unité d'aspiration (15) et introduites dans le liquide circulant dans le circuit principal (5), et
le procédé est exécuté automatiquement ou semi-automatiquement par une unité de commande présente dans un dispositif de mélange, ladite unité de commande étant configurée pour piloter les actionneurs nécessaires, notamment des pompes et des vannes, et pour lire les valeurs de mesure nécessaires provenant de capteurs.

13. Procédé selon la revendication 12, **caractérisé en ce que** le solvant ou la solution en cours de formation est introduit depuis le circuit principal (5) via la conduite de dilution (11) dans le récipient de matière première (18), est mélangé aux matières premières et est aspiré à travers la conduite d'aspiration (16) conjointement avec les matières premières.

14. Procédé selon la revendication 12 ou 13, **caractérisé en ce que** la préparation de la solution s'effectue de manière autorégulée à l'aide de la chambre de mélange fin (48_1, 48_2) disposée dans le circuit principal et de l'élément de retenue qu'elle contient.

15. Utilisation d'un système selon la revendication 10 ou 11 pour la préparation d'une solution médicale et de préférence d'une solution de concentré de dialyse.
